## Europäisches Patentamt

## European Patent Office

## Office européen des brevets

(19)

(11) Veröffentlichungsnummer : **0 025 508**
**B1**

(12) **EUROPÄISCHE PATENTSCHRIFT**

(45) Veröffentlichungstag der Patentschrift :
03.08.83

(21) Anmeldenummer : 80104716.8

(22) Anmeldetag : 09.08.80

(51) Int. Cl.³ : **C 07 G  7/00**, A 61 K 39/00,
B 01 D 15/00

(54) Verfahren zur Herstellung der dritten Komponente des Komplements aus menschlichem Blutplasma.

(30) Priorität : 16.08.79 DE 2933015

(43) Veröffentlichungstag der Anmeldung :
25.03.81 Patentblatt 81/12

(45) Bekanntmachung des Hinweises auf die Patenterteilung : 03.08.83 Patentblatt 83/31

(84) Benannte Vertragsstaaten :
AT BE CH DE FR GB IT LI NL SE

(56) Entgegenhaltungen :
US A 4 075 193

Chemical abstracts Band 86, Nr. 17, 25. April 1977 Columbus, Ohio, USA A. WICHMAN et al. « Purification of human immunoglobulin M by affinity chromatography on protamine-Sepharose » Seite 368, Spalte 1, Abstract Nr. 119050 g

Chemical Abstracts Band 90 Nr. 9, 26. Februar 1979 Columbus, Ohio, USA M. MATTAUSCH « Purification of neutral proteases from leucocytes on Protamine-Sepharose » Seite 187, Spalte 2, Abstract Nr. 68325y

(73) Patentinhaber : BEHRINGWERKE AKTIENGESELLSCHAFT
Postfach 1140
D-3550 Marburg/Lahn (DE)

(72) Erfinder : Becker, Udo, Dr.
Schmaedelstrasse 17
D-8000 München 60 (DE)
Erfinder : Heimburger, Norbert, Dr.
Sonnenhang 10
D-3550 Marburg/Lahn (DE)

(74) Vertreter : Meyer-Dulheuer, Karl-Hermann, Dr. et al
HOECHST Aktiengesellschaft Zentrale Patentabteilung Postfach 80 03 20
D-6230 Frankfurt/Main 80 (DE)

# Verfahren zur Herstellung der dritten Komponente des Komplements aus menschlichem Blutplasma

Die Erfindung betrifft ein Verfahren zur Isolierung und Reinigung der dritten Komponente des Komplements (C3) aus Blutplasma und anderen Lösungen, die das C3 enthalten.

Das Komplementsystem ist Teil der immunologischen Abwehr des Körpers gegen Fremdstoffe. Dringen Fremdstoffe, z. B. Viren oder Bakterien in den Körper ein, so wird die immunologische Abwehr mobilisiert, in deren Folge auch das Komplementsystem aktiviert wird. Das Komplementsystem besteht im wesentlichen aus neun Komponenten, die alle Proteine sind und mit C1 bis C9 bezeichnet wurden. Eine zentrale Rolle nimmt C3 ein, das mit ca. 120 mg/dl im normalen Blutplasma vorkommt. Es wird im Ablauf der Komplementaktivierung durch einen aus C2 und C4 bestehenden Komplex enzymatisch gespalten. Das dabei entstehende Fragment C3b bindet sich an die abzuwehrende Fremdzelle und leitet die Zerstörung der Zellmembran durch die weiteren Komplementkomponenten C5 bis C9 ein.

Während ein angeborener Mangelzustand von C3 selten vorkommt, sind pathologische Prozesse, die mit einem Verbrauch von C3 einhergehen, sehr häufig (s. A.B. LAURELL : Komplementfaktoren in der Labormedizin, Laboratoriumsblätter 27, 89-98 (1977). Die größte Gruppe stellen alle Arten von akuten und chronischen bakteriellen Infektionen dar, z. B. Streptokokken-Infektionen, E. coli und Meningokokken-Sepsis. Ebenso tritt bei vielen entzündlichen rheumatischen Erkrankungen ein Verbrauch von C3 ein, ebenso wie bei Autoimmunerkrankungen des Typs lupus erythematodes oder immunhämolytische Anämie.

Der Bestimmung von C3 kommt daher diagnostisch eine große Bedeutung zu. Ein therapeutischer Einsatz von C3 ist jedoch derzeit noch nicht absehbar, u. a. deshalb, weil bisher kein wirtschaftliches Verfahren zur Gewinnung von C3 zur Verfügung steht. Die bisherigen, dem Stand der Technik entsprechenden Gewinnungsmethoden sind außerordentlich aufwendig und umfassen zahlreiche Fällungs-, Chromatographie- und Elektrophoreseschritte (H.-J. MÜLLER-EBERHARD, Methods in Immunology and Immunochemistry, Band IV, S. 212-217, (1977)). Die Ausbeute an C3 erreicht dabei höchstens 10 %.

Es wurde nun überraschenderweise gefunden, daß eine Anreicherung von C3 gemäß einem sehr einfachen Verfahren erzielbar ist. Dieses beruht auf der Affinitätschromatographie mittels Protamin als C-affinem Material. Lt. Chem. Abstr. Bd. 90 (1979) Ref. 68325y wurde Protamin-Sepharose ® 4B zur Gewinnung eines Proteinase-Inhibitors eingesetzt. Ein solcher Inhibitor hat jedoch keine Beziehung zum C3-Protein.

Gegenstand der Erfindung ist ein Verfahren zur Herstellung bzw. Anreicherung der dritten Komponente des Komplements (C3) aus dessen Lösungen, dadurch gekennzeichnet, daß man die Lösung in Kontakt bringt mit an einen wasserunlöslichen Träger gebundenem Protamin, danach dieses von der Lösung abtrennt und schließlich das C3 von dem trägergebundenen Protein wieder ablöst. Ein bevorzugt verwendetes Protamin ist vor allem dessen Salzform, vorzugsweise das Protaminsulfat. Günstig für die Zwecke der Erfindung hat sich Salminsulfat, das ist das Protaminsulfat aus Salm, erwiesen.

Als Trägermaterialien, die in wässrigen Systemen praktisch unlöslich sind, eignen sich partikuläre Substanzen, wie sie allgemein für die Träger der Affinitätschromatographie verwendet werden. Es sind dies : Cellulose, SEPHADEX ®, Polyacrylamid und Ionenaustauscher auf Basis dieser 3 Verbindungen.

Bevorzugt wird Agarose. An diese Trägersubstanzen wird nach an sich bekannten Verfahren das Protamin gebunden. Danach steht das Adsorbens für die Affinitätschromatographie von C3 zur Verfügung. Dies kann nun entweder als Säulenfüllung verwendet oder im sogenannten Batch-Verfahren für die Adsorption des C3 eingesetzt werden.

Diese Bedingungen für die Adsorption des C3 aus dessen wässriger Lösung, vorzugsweise aus Blutplasma, sind wie folgt :

Die C3-haltige Lösung, vorzugsweise frisches Human-Citrat-Plasma mit einem Gehalt an C3 von ca. 120 mg/dl, wird soweit verdünnt, daß die Adsorption des C3 an das C3-affine Material erfolgt. Bevorzugt wird mit dest. Wasser auf die Leitfähigkeit einer etwa halbphysiologischen Kochsalzlösung verdünnt. Mit dieser Lösung wird eine mit dem C3-affinen Material, vorzugsweise mit trägergebundenem Protamin gefüllte Säule mit Sepharose als Trägermaterial beschickt, die bereits mit halbphysiologischer Kochsalzlösung equilibriert wurde.

Die Adsorption, nachfolgende Waschung und Elution kann auch im Batch-Verfahren erfolgen ; es ist auch empfehlenswert, die Adsorption im Batch-Verfahren durchzuführen und die anschließende Elution in der Säule. Das Batch-Verfahren empfiehlt sich vor allem für die Herstellung in größerem Maßstab, die Elution in der Säule speziell für die Gewinnung von hochgereinigtem Material, da in Verbindung mit der Säulenelution ein Puffergradient verwendet werden kann, der gewährleistet, daß man die Elutionsbedingungen genau trifft.

Bei Verwendung der Säulen-Elution wird der Ausfluß des Chromatographie-Rohres an die Durchfluß-Küvette eines automatisch registrierenden Photometers angeschlossen und die optische Dichte bei 280 nm vermessen ; wenn diese nach Auftragen der Substanz wieder die Grundlinie erreicht hat, wird z. B. die Protamin-Sepharose mit Kochsalzlösung gewaschen, zunächst mit halbphysiologischer, dann mit physiologischer NaCl und so lange, bis die optische Dichte zur Basis zurückgekehrt ist.

Die Elution erfolgt mit wässrigen Lösungen, deren Ionenstärke der von NaCl-Lösungen von 0,3 bis 0,5 mol/l entsprechen. Für die Gewinnung eines C3-Konzentrates zur therapeutischen Anwendung empfiehlt sich die Elution im Batch-Verfahren mit einem Puffer ≥ 0,38 mol NaCl/l.

Es wird jeweils diejenige Fraktion gewonnen, welche das C3 enthält. Der Nachweis des C3 erfolgt mit den hierfür üblichen Methoden, vorzugsweise mit einem immunologischen Nachweisverfahren durch Verwendung eines mehr oder weniger spezifischen Anti C3-Serums.

Für die Herstellung eines hochgereinigten C3-Antigens, z. B. für die Immunisierung, ist die Elution in der Säule angezeigt, unter Verwendung eines linearen Puffergradienten, hergestellt aus gleichen Volumina unterschiedlich konzentrierter Kochsalzlösung, z. B. einer 0,9 %igen physiologischen und einer 0,5 molaren Kochsalzlösung. Die bei der Chromatographie anfallenden Fraktionen können in der Polyacrylamid-Gel-Elektrophorese nach ZWISLER, O. und H. BIEL, Z. klin. chem. *4*, 58 (1966), charakterisiert und danach gesammelt werden. Erfahrungsgemäß erscheint die Fraktion mit höchstem Reinheitsgrad in dem Eluat, das mit 0,38 mol/l NaCl desorbiert werden kann. Die Fraktionen höchster Reinheit werden vereint und entsprechend ihrem Verwendungszweck konzentriert, dialysiert und ggf. auch lyophilisiert. Sie enthalten nach Untersuchungen mit spezifischen Antiseren das C3 in hochgereinigter und nativer Form.

Die hier beschriebene Methode erreicht Ausbeuten bis zu 90 % in einem einzigen Verfahrensschritt. Die Ausbeute hängt dabei von dem gewünschten Reinheitsgrad ab, der zwischen 50 und 80 % liegt. Gewünschtenfalls kann das erfindungsgemäße Verfahren wiederholt und/oder mit anderen proteinchemischen Verfahren kombiniert werden, um ein Reinprodukt C3 zu erhalten.

C3 ist ein bekanntermaßen labiles Protein, das leicht zu den Fragmenten C3a und C3b und dieses weiter zu C3c und C3d degradiert wird. Das Ausmaß der erfolgten Degradation läßt sich durch die Technik der zweidimensionalen Immunelektrophorese feststellen. Das nach dem erfindungsgemäßen Verfahren hergestellte C3 befindet sich nach dieser Technik in einem nativen, nicht degradierten Zustand.

Die Erfindung wird durch das nachfolgende Beispiel erläutert.

Beispiel

a) Herstellung des spezifischen Adsorbens :
Agarose (Sepharose 4 B, Pharmacia Fine Chemicals, Uppsala, Schweden) wird nach dem Verfahren von AXEN, PORATH und ERNBACK, Nature *214*, 1302-1304 (1967) mit Bromcyan aktiviert.

Eine Menge dieser Agarose, welche einem Trockengewicht von 8 g entspricht, wird in 250 ml 0,1 molarer NaHCO₃ — Lösung vom pH 9 aufgenommen und 0,8 g Salminsulfat (Firma Roth, Karlsruhe) gelöst in 50 ml derselben Lösung werden eingerührt. Die Mischung wird unter Schütteln 16 Stunden bei Raumtemperatur inkubiert und das Gel anschließend mit Wasser und mit sauren und basischen Puffern gewaschen. Vor Gebrauch erfolgt Äquilibrierung mit 1 : 2 verdünnter physiologischer Kochsalzlösung.

b) Anreicherung bzw. Gewinnung von C3

Eine Glassäule der Abmessung 2,5 × 22 cm wird mit der Salminsulfat beladenen und mit 1 : 2 verdünnter physiologischer Kochsalzlösung äquilibrierten Agarose gefüllt. 20 ml mit Citrat, ACD-Lösung oder EDTA antikoaguliertes menschliches Blutplasma werden mit destilliertem Wasser auf das doppelte Volumen verdünnt und auf die Säule aufgetragen. Das aus der Säule austretende Eluat wird kontinuierlich photometrisch bei 280 nm gemessen und von einem Schreiber registriert. Es wird mit 200 ml der halbkonzentrierten physiologischen Kochsalzlösung gewaschen und die Ionenstärke dann auf physiologische Konzentration erhöht. Nach weiteren 200 ml wird ein linearer Gradient, bestehend aus je 200 ml physiologischer Kochsalzlösung und 0,5 molarer Kochsalzlösung angelegt und das Säuleneluat in 5 ml Fraktionen gesammelt. Der Schreiber registriert vier Proteinfraktionen, die bei den Ionenstärken 0,30, 0,38, 0,45 und 0,5 ihr jeweiliges Maximum besitzen (Abb. 1). Die immunologische Analyse ergibt, daß die Fraktion bei 0,38 C3 ist. Die Fraktion wird gesammelt und analysiert.

Die quantitative Immunelektrophorese nach LAURELL, C.-B., Analyt. Biochem. *15*, 45 (1966) ergibt eine Ausbeute von 89 %.

Zur Beurteilung des Reinheitsgrades werden die Fraktionen, die im jeweiligen Maximum liegen, in der Polyacrylamid-Gel-Elektrophorese nach ZWISLER, O. und H. BIEL, Z. klin. chem. 4, 58 (1966), aufgetrennt (Abb. 2). C3 erscheint einheitlich, die als Verunreinigung auftretenden Proteine stammen aus den jeweils angrenzenden Fraktionen. Gewünschtenfalls können diese noch durch eine Wiederholung der Gradientenchromatographie an Salmin-Agarose unter den genannten Bedingungen von C3 abgetrennt werden.

**Ansprüche**

1. Verfahren zur Herstellung bzw. Anreicherung der dritten Komponente des Komplements aus menschlichem Blutplasma (C3) aus dessen Lösungen, dadurch gekennzeichnet, daß man die Lösung in Kontakt bringt mit an einen wasserunlöslichen Träger gebundenem Protamin oder dessen Salz, danach dieses von der Lösung abtrennt und schließlich das C3 von dem trägergebundenen Protamin wieder ablöst.

2. Verfahren nach Anspruch 1, dadurch gekennzeichnet, daß der wasserunlösliche Träger Agarose ist.

**Claims**

1. A process for preparing or enriching the third component of the complement of human plasma (C3) from solutions containing it, which comprises contacting said solutions with protamine or a salt thereof, which is fixed to a water-insoluble carrier, separating said material from said solutions and detaching C3 from said material.

2. The process of claim 1, wherein the water-insoluble carrier is agarose.

## Revendications

1. Procédé pour la préparation de, ou l'enrichissement, à partir de ses solutions, en la troisième composante du complément du plasma sanguin humain (C3), caractérisé en ce qu'on met la solution en contact avec une protamine ou son sel, fixé sur un support insoluble dans l'eau, puis on sépare celui-ci de la solution et on sépare le C3 de la protamine fixée sur le support.

2. Procédé selon la revendication 2, caractérisé en ce que le support insoluble dans l'eau est l'agarose.